# EUROPEAN PATENT APPLICATION

(11) **EP 2 380 528 A1**
(43) Date of publication of application: **26.10.2011**
(21) Application number: 10731027.8
(22) Date of filing: 19.01.2010
(51) Int. Cl.: A61F 2/82

(54) **MEDICAMENT ELUTING APPARATUS WITH MICRO-HOLE STRUCTURE CAPABLE OF STORING AND RELEASING MULTIPLE MEDICINES AND PREPARATION METHOD**

(30) Priority: 19.01.2009 CN 200910077070
(71) Applicant: Beijing Target Technology Co., Ltd, Changping Tech. Zone, Beijing 102200 (CN)
(72) Inventor: ZHANG, Yuxin, Beijing 102200 (CN); ZHAO, Lixiao, Beijing 102200 (CN); PU, Zhongjie, Beijing 102200 (CN)
(74) Representative: Richter, Werdermann, Gerbaulet & Hofmann
(86) International application number: PCT/CN2010/000080
(87) International publication number: WO 2010/081393

(57) **Abstract**

A medicament eluting apparatus with a micro-hole structure capable of storing and releasing multiple medicines and preparation method thereof are provided. The medicament eluting apparatus includes an apparatus body (10) and several holes (40) on the apparatus body (10), and multiple medicines (20, 30) are provided in the holes (40) and on the surface of the apparatus body (10) which contains no polymers. The multiple medicines (20, 30) are selected from medicine therapeutic agents, carrier therapeutic genes and bioactive substances, wherein one or two of the medicine therapeutic agents is/are taxol and/or probucol. The preparation method of the medicament eluting apparatus includes: 1) the holes (40) prepared on the surface of the apparatus body (10); 2) the multiple medicines (20, 30) coated in the holes (40) and on the surface of the apparatus body (10).

## Description

### Technical Filed

The invention relates to an implantable medical apparatus and the preparation method thereof. Specifically, the invention relates to a medicament eluting apparatus with micro-hole structure capable of storing and releasing multiple medicines and the preparation method thereof.

### Background Art

The medicament eluting apparatus is one kind of implantable medical apparatuses, including stents, catheters, guide wire, cardiac pacemaker, heart valves, surgical implant materials, hard tissue implants and other implantable medical apparatuses capable of releasing medicines. Taking vascular stent as an example, early vascular stent was bare stent, including stainless steel, titanium, cobalt alloys and nickel-titanium shape memory alloy, etc. But they had a high restenosis rate of above 30%. After using the first-generation drug-eluting stent (drug-loaded polymer stent), the restenosis rate was greatly reduced. The eluting drugs include heparin, corticosteroids, agent resistant to cell proliferation and so on. These drugs were fused to synthetic polymers and coated on the stent surface, and they can achieve an effective blood concentration on the local lesions and steadily release for several weeks or months, thus inhibiting immune and mitosis, proliferation of vascular smooth muscle cells and intima thickening, so as to prevent restenosis after PCI treatment. However, the restenosis rate of the first-generation drug-eluting stents remains about 5%, some even reach 10% or more. Furthermore, the first-generation drug-eluting stents are not effective for treatment of some complex lesions, such as CTO with complex causes of (chronic total occlusion).

There are a variety of factors which can cause restenosis, for example, endothelial injury during interventional treatment, inflammatory response to the foreign matters including polymers, excessive proliferation of neointima, negative vascular remodeling, rupture of the plaque and thrombosis on the lesion and so on. The restenosis of the target vascular lesions of different patients may be associated with one or more factors mentioned above, more often, caused by two or more factors. More complex lesions are usually caused by more factors. Hence, a desired drug should be able to resist restenosis relating to various factors.

However, different anti-restenosis drugs play their limited roles, because the effects and mechanisms of the coated stents are different.

As coated with a single drug, the first-generation stent has many limitations in treatment effects. The Heparin-coated Stent can reduce the incidence of subacute stent thrombosis and inhibit the neointimal proliferation. The clinical nonrandomized controlled trials has proved that the Heparin-Coated Stent is safe for use on human with a good biocompatibility. Compared with bare stents, the Heparin-Coated Stent can also reduce the incidence of subacute thrombosis. However, after more than 6 months of long-term follow-up, it is found that Heparin-Coated Stent has no significant difference from bare stent in major adverse cardiac events (MACE) and stent restenosis. Thus, the long-term effect of heparin-coated stent in reducing MACE and stent restenosis is unsatisfactory.

A single drug (such as rapamycin, etc.)-containing polymer-coated stent is another most widely used stent, it inhibits restenosis depending on the drug effects of strong immunosuppression and anti-proliferation. All the clinical nonrandomized controlled trials have shown that the stent can effectively inhibit restenosis in a short term, but it is not perfect: some studies indicate that the stent fails to form endothelialization in a short term, which means it has a higher risk of thrombosis than that of the bare stent. Recent studies even showed that the endothelialization of the stent is still incomplete after implanting the single drug-containing polymer-coated stent for two years.

Furthermore, some other studies show that there is a late catch-up phenomenon of restenosis after implanting the single drug-containing polymer-coated stent for a long time. Relevant research analysis found that, the polymer may be the main reason of delayed endothelialization and late catch-up of restenosis, and it is also significantly connected with the singleness of the drug. Firstly, the long-existing polymer leads to a persistent inflammatory response; secondly, the drug only inhibiting restenosis inhibits not only endothelial neointima but also the growth of the endothelium, and this single drug has little effect on the restenosis caused by a variety of complex factors.

In order to solve the singleness problem of drug, it is disclosed in recent patents (US 2008/0241215 A1, US 2008/0172124 A1, etc.) that on the basis of the first-generation DES antioxidant drugs are added to prevent further restenosis and some adverse reactions. However, these patents all use the polymer to control the release of the two drugs, and the two drugs can be blended into the same or different polymer. After implantation, although the stent can achieve better effects in inhibiting restenosis, the increased amount of polymer will also bring more serious adverse reactions.

Thus, on one hand, the existing single drug-containing polymer-coated stent can only have a limited inhibitory effect or even just delaying effect on the incidence of restenosis, and can not simultaneously reduce other adverse reactions, such as endothelialization and thrombosis, since it often carries a single drug of anti-cell proliferation. On the other hand, although the existence of the polymer can better control the release of drugs, the persistent inflammation caused by the polymer will be more fatal.

Therefore, in order to overcome the defects of the existing single drug-containing polymer-coated stents, the present invention provides a non-polymeric medicament eluting apparatus with micro-hole structure capable of storing and releasing multiple medicines. Combining a variety of medicaments to act on the target lesion can inhibit neointimal proliferation, thus promoting faster growth of endothelial and reduce the adverse reaction. Thus the medicament eluting stent having no polymers with micro-hole structure is more reliable than the drug-containing polymer-coated stent, and it inhibits neointimal proliferation through different mechanisms after being treated with multiple drugs. The medicament eluting stent can treat more lesion types, and it promotes endothelium to grow faster than using single drug, so as to permanently inhibit the incidence of restenosis.

Meanwhile, based on the above-mentioned advantages, the medicament eluting apparatus according to the present invention can also be applied to various implantable medical apparatuses other than the stent, such as catheters, guide wire, cardiac pacemaker, cardiac valves, appendage, surgical implant materials and hard tissue implants and so on. It can sustainably release multiple medicines to ensure an effective local blood concentration on the lesion area for a long term, thus achieving good treatment and prognosis effects.

### Summary of the Invention

In view of the above mentioned problems, the object of the present is to provide a medicament eluting apparatus with micro-hole structure capable of storing and releasing multiple medicines and preparation method thereof.

In order to achieve the above object, the following technical solution is provided for the medicament eluting apparatus with a micro-hole structure capable of storing and releasing multiple medicines:

The medicament eluting apparatus according to the present invention includes an apparatus body and a hole structure for release of multiple medicines, in which the hole structure for medicine release refers to a number of holes in the apparatus body and the multiple medicines present in the holes and on the surface of the apparatus body (referred to as multiple medicines stored on the apparatus surface), wherein the holes are nano-scale or micron-scale, the mean value of the hole size (d) and hole depth (h) vary in the range of 1 nm to 500 µm.

The said holes are mono-size or bimodal-size or multimodal-size holes in micron-scale or nano-scale, that is, with a number of n, the holes are micron-scale or nano-scale holes distributed in even size, or the holes are micron-scale or nano-scale holes distributed in uneven size and have two or more statistic mean values of the hole size or the hole depth.

The said mono-size holes in nano-scale is selected from any one of the group consisting of: nano-scale holes with even size, nano-scale holes with big size (the size is in the range of 500 nm to 1000 nm), nano-scale holes with small size(the size is in the range of 50 nm to 500 nm), deep holes in nano-scale (the depth is in the range of 200 nm to 1000 nm) and shallow holes in nano-scale (the depth is in the range of 20 nm to 200 nm).

The said bimodal-size holes in nano-scale include two different sizes of holes: big size and small size holes, or include two holes with different depth: deep holes and shallow holes, or include two kinds of nano-scale holes with different hole size and hole depth. The active medicines are loaded in various nano-scale holes (including various big size nano-scale hole and/or small size nano-scale hole and/or deep nano-scale hole and/or shallow nano-scale hole).

The said multimodal-size holes in nano-scale include three or more nano-scale holes with different hole sizes and hole depths as follows: big size nano-scale holes, small size nano-scale holes, deep nano-scale holes and shallow nano-scale holes. The active medicines are loaded in various big size nano-scale holes and/or small nano-scale holes and/or deep nano-scale holes and/or shallow nano-scale holes.

The said nano-scale holes with even size, nano-scale holes with big size, nano-scale holes with small size, deep holes in nano-scale and shallow holes in nano-scale are presented in opening, semi-opening, closed, separated, inter-connected, inter-embedded type, or nested type in which a large hole comprises small holes.

The said multiple medicines provided in the holes and on the surface of the apparatus body refer to two or more medicines selected from one or more of the group consisting of: medicine therapeutic agents, carrier therapeutic genes and bioactive substances.

The said medicine therapeutic agents are selected from one or more of the group consisting of: heparin, aspirin, hirudin, colchicine, antiplatelet GPIIb/IIIa receptor antagonist, methotrexate, purines, miazines, plant alkaloids and epothilones, series compounds of Tripterygium *Wilfordii*, antibiotics, hormones, antibody medicines for cancer treatment, cyclosporin, tacrolimus and homologues thereof (FK506), 15-deoxyspergualin, mycophenolate mofetil (MMF), rapamycin and derivatives thereof, FR 900520 (a strain of Streptomyces), FR 900523 (a strain of Streptomyces), NK 86-1086, daclizumab, depsidomycin (valeramide), kanglemycin C , spergualin, prodigiosin 25-c, tranilast, myriocin, FR 651814, SDZ214-104, cyclosporine C, bredinin, mycophenolic acid, brefeldin A, WS9482, glucocorticosteroid, tirofiban, abciximab, eptifibatide, taxol, actinomycin-D, AS₂O₃, 17β-estradiol, trapidil, hexadecadrol, probucol and derivatives thereof.

The said carrier therapeutic genes are selected from one or more of the group consisting of: cell, virus, DNA, RNA, virus carrier and nonviral carrier.

The said bioactive substances are selected from one or more of the group consisting of: cell, yeast, bacteria, protein, peptide and hormone.

Preferably, the multiple medicines are taxol and/or probucol.

The said apparatus body includes stent, catheters, guide wire, cardiac pacemaker, cardiac valve, surgical implant material, hard tissue implants, and nonmetal medical apparatus of ceramic, organic polymer, inorganics, metal oxide matrix; the said stent includes balloon expanded stent, self-expanded stent, vascular stent, non-vascular stent, stent with medical stainless steel, nickel-titanium shape-memory alloy, cobalt alloy, pure titanium, titanium alloy and tantalum, titanium alloy or gold matrix which has good biocompatibility, and the wire-braided, pipe-laser-cutting, mould-casting and welding stent.

The said apparatus body may further comprise an outermost film layer of protein, polysaccharides, medicine therapeutic agents or other materials, which can be absorbed by an organism.

The said multiple medicines stored on the apparatus surface can be obtained by coating a mixture of multiple medicines on the surface of the apparatus body.

The said multiple medicines stored on the apparatus surface can be obtained by firstly coating several medicines and then coating the remained medicines on the surface of the apparatus body.

The said multiple medicines stored on the apparatus surface can be obtained by firstly coating several medicines and then coating all the medicines on the surface of the apparatus body.

The said multiple medicines stored on the apparatus surface can be obtained by firstly coating a mixture of multiple medicines and then coating several medicines thereof on the surface of the apparatus body.

The said multiple medicines stored on the apparatus surface can be obtained by firstly coating several medicines on the inner surface of the apparatus body and then coating the other medicines on the rest surfaces of the apparatus body.

The said multiple medicines stored on the apparatus surface can be obtained by firstly coating the mixture of all the medicines on the inner surface of the apparatus body and then coating several medicines thereof on the rest surface of the apparatus body.

The said multiple medicines stored on the apparatus surface can be obtained by firstly coating several medicines on part of surface of the apparatus body and then coating other medicines on the rest part of the surface of the apparatus body.

The said multiple medicines stored on the apparatus surface can be obtained by firstly coating several medicines on the upper part of the apparatus and then coating other medicines or all the medicines or several medicines thereof on the lower part of the apparatus.

The said multiple medicines stored on the apparatus surface can be obtained by firstly coating several medicines on the front part of the apparatus and then coating other medicines or all the medicines or several medicines thereof on the hind part of the apparatus.

The said multiple medicines stored on the apparatus surface refer to two or more medicines. Coating two medicines is taken as an example:

The said two medicines stored on the apparatus surface can be obtained by coating the mixture of the two medicines on the surface of the apparatus body.

The said two medicines stored on the apparatus surface can be obtained by firstly coating one medicine and then coating the other medicine on the surface of the apparatus body.

The said two medicines stored on the apparatus surface can be obtained by firstly coating the mixture of the two medicines and then coating one medicine thereof on the surface of the apparatus body.

The said two medicines stored on the apparatus surface can be obtained by firstly coating one medicine on the inner surface of the apparatus body and then coating the other medicine on the rest surfaces of the apparatus body.

The said two medicines stored on the apparatus surface can be obtained by firstly coating the mixture of the two medicines on the inner surface of the apparatus body and then coating one medicine thereof on the rest surfaces of the apparatus body.

The said two medicines stored on the apparatus surface can be obtained by firstly coating one or two medicines on part of, for example, the upper part (half part) of the apparatus, and then coating the other medicine or one of the two medicines on another part of, for example, the lower part (the other half part) of the apparatus.

The said two medicines stored on the apparatus surface can be obtained by firstly coating one medicine (or two medicines) on part of, for example, the front part (half part) of the apparatus, and then coating the other medicine (or one of the two medicines) on another part of, for example, the hind part (the other half part) of the apparatus.

One of the two medicines stored on the apparatus surface can be taxol or probucol.

The said two medicines stored on the apparatus surface can be obtained by firstly coating one medicine and then coating taxol or probucol on the surface of the apparatus body.

The said two medicines stored on the apparatus surface can be obtained by firstly coating taxol or probucol and then coating the other medicine on the surface of the apparatus body.

The said two medicines stored on the apparatus surface can be obtained by firstly coating one medicine on the inner surface of the apparatus body and then coating taxol or probucol on the rest surface(s) of the apparatus body.

The said two medicines stored on the apparatus surface can be obtained by firstly coating taxol or probucol on the inner surface of the apparatus body and then coating the other medicine on the rest surface(s) of the apparatus body.

The said two medicines stored on the apparatus surface can be probucol and taxol.

The said two medicines stored on the apparatus surface can be obtained by firstly coating taxol and then coating probucol on the surface of the apparatus body.

The said two medicines stored on the apparatus surface can be obtained by firstly coating probucol and then coating taxol on the surface of the apparatus body.

The said two medicines stored on the apparatus surface can be obtained by firstly coating one medicine on part of the surface, for example, the inner surface of the apparatus body, and then coating probucol on the rest surface(s).

The said two medicines stored on the apparatus surface can be obtained by firstly coating probucol on the inner surface of the apparatus body, and then coating taxol on the rest surface(s).

One of the two medicines stored on the apparatus surface can be rapamycin.

The said two medicines stored on the apparatus surface can be obtained by firstly coating rapamycin on the inner surface of the apparatus body, and then coating the other medicine on the rest surface(s).

The said two medicines stored on the apparatus surface can be obtained by firstly coating one medicine on the inner surface of the apparatus body, and then coating rapamycin on the rest surface(s).

When the said two medicines stored on the apparatus surface are rapamycin and probucol, the way of coating the medicines can be: firstly coating rapamycin and then probucol; or firstly coating probucol and then rapamycin; or coating a mixture of rapamycin and probucol; or firstly coating rapamycin on the inner surface of the apparatus body then probucol on the rest surface(s).

The said more than two medicines stored on the apparatus surface can be obtained by firstly coating two medicines and then coating other medicines, or coating the medicines in turn, or coating a mixture of the multiple medicines.

One of the multiple medicines stored on the apparatus surface can be taxol.

One of the multiple medicines stored on the apparatus surface can be probucol.

One of the multiple medicines stored on the apparatus surface can be heparin.

One of the multiple medicines stored on the apparatus surface can be rapamycin.

One of the multiple medicines stored on the apparatus surface can be tacrolimus.

One of the multiple medicines stored on the apparatus surface can be β-estradiol.

The coating manner of the said more than two medicines stored on the apparatus surface is the same as that of the two medicines. Coating three medicines is taken as an example:

The said three medicines stored on the apparatus surface can be obtained by firstly coating a mixture of two medicines and then coating the third medicine on the surface of the apparatus body.

The said three medicines stored on the apparatus surface can be obtained by coating the three medicines in turn on the surface of the apparatus body.

The said three medicines stored on the apparatus surface can be obtained by coating one different medicine on the different part (e.g. upper part and lower part, inner part and outer part, the adjacent strip areas and so on.) of the surface of the apparatus body, respectively.

The said three medicines stored on the apparatus surface can be obtained by firstly coating one different medicine on the different part (e.g. upper part and lower part, inner part and outer part, the adjacent strip areas and so on) of the surface of the apparatus body, and then coating another medicine (or a mixture of the other two medicines).

One of the three medicines stored on the apparatus surface can be taxol.

One of the three medicines stored on the apparatus surface can be probucol.

One of the three medicines stored on the apparatus surface can be heparin.

One of the three medicines stored on the apparatus surface can be rapamycin.

One of the three medicines stored on the apparatus surface can be tacrolimus.

One of the three medicines stored on the apparatus surface can be β-estradiol.

Preferably, the way of coating three medicines can be: firstly coating a mixture of taxol and probucol on the surface and in the micro-holes of the apparatus, then coating a layer of heparin on the same. Taxol and probucol can play the role of inhibition of restenosis, and heparin can play the role of resistance of thrombosis.

For coating four or more than five medicines, specifically, the way of coating can be : firstly coating a mixture of taxol and probucol on the surface and in the micro-holes of the apparatus, then dip-coating active substances such as antibodies or peptides on the surface, finally coating a layer of heparin. Taxol and probucol can play the role of inhibition of restenosis; antibodies, peptides and other active substances can promote the growth of endothelial cells; and heparin can resist thrombosis.

The dose of said multiple medicines stored on the apparatus surface is therapeutic dose or less, which is well known to the skilled person in the art, such as the therapeutic dose of probucol is about 2µg to 1000µg (dose per stent); the therapeutic dose of rapamycin is about 0.2µg to 1200µg (dose per stent); the therapeutic dose of heparin is about 0.2µg to 1200µg (dose per stent); the therapeutic dose of taxol is about 2µg to 1000µg (dose per stent); the therapeutic dose of tacrolimus is about 0.2µg to 1200µg (dose per stent); the therapeutic dose of β-estradiol and other hormones is about 0.2µg to 1200µg (dose per stent).

The coating process is selected from one or more of the group consisting of: spraying, immersing, dipping, electrostatic coating, sol-gel coating, and supercritical liquid coating.

The present invention also provides a method for preparation of the medicament eluting apparatus with a micro-hole structure capable of storing and releasing multiple medicines, the method mainly comprises preparing a hole structure for releasing medicines on the implantable medical apparatus body, including the steps of:
(1) preparing holes on the surface of the apparatus body with one or more methods selected from the group consisting of: corrosion with acid solution, anodic oxidation, microarc oxidation and microarc nitridation or combinations thereof;
(2) coating multiple medicines in the holes and on the surface of the apparatus body through the methods comprising spraying or dipping.

The said corrosion with acid solution refers to immersing the equipment body material in the etching solution at the temperature of 0 to 100 °C, preferably, the said etching solution can be hydrochloric acid with a concentration of 1 to 38%, or a mixed acid solution containing 1 to 38% hydrochloric acid and 1 to 98 % sulfuric acid, or hydrofluoric acid with a concentration of 1 to 30%, or a mixed acid solution mixed in any proportion which contains one or more of hydrochloric acid, sulfuric acid and hydrofluoric acid with any concentration; the corrosion time is controlled in 1min to 480h.

The said anodic oxidation refers to connecting the apparatus body material as the anode with the anode of a pulse power supply, connecting the sheet metal made of titanium, magnesium, aluminum, iron, zinc, copper, gold, silver, platinum and alloys thereof as the cathode with the cathode of a pulse power supply, placing the apparatus body and the cathode sheet metal into the electrolyte simultaneously, wherein, preferably, the electrolyte is hydrochloric acid solution with a concentration of 1 to 38% or sulfuric acid solution with a concentration of 1 to 98%, the current is set to 0.01 to 30A, the frequency is 25 to 3000 Hz, and the time is 1 to 20min.

Mono-size nano-scale holes can be directly prepared in the apparatus body raw material; or firstly the mono-size nano-scale holes are directly prepared in the apparatus body raw material by corrosion with acid solution, and then the multimodal-size nano-scale complex holes are prepared by anodic oxidation or the combination of micro-arc oxidation and microarc nitridation;

The method for preparation of the medicament eluting apparatus according to the present invention further comprises dissolving the multiple medicines respectively or mixedly in a solvent to prepare pharmaceutical solution prior to the step (2).

The said medicines solution is obtained by dissolving the medicines in a solvent, wherein the weight ratio of the medicines and the solvent is 1:10 to 1:10000;

Preferably, the solvent is selected from one or more of the group consisting of: ethanol, saline, acetone, dimethyl sulfoxide, isopropyl acetate, ethyl acetate, ethyl carbonate, methyl acetate, tetrahydrofuran, isobutyl alcohol, propyl acetate and butanone or the mixed solvents thereof.

The coating process is selected from one or more of the group consisting of: spraying, dipping and dipping in vacuum and so on; after coating, a medicine layer is formed when the solvent is evaporated.

Wherein the coating can be carried out as follows: the apparatus body is installed in the spraying machine, the above prepared mixed solution of activity medicines, polymer and organic solvent are sprayed in the holes of the surface of the apparatus body, part of mixed solution is adhered to the surface of the apparatus body; the said dipping is to immerse the apparatus body in the medicine solution; said dipping in vacuum is to vacuum the environment around the apparatus and medicines solution, so as to make the medicines more easily attached to the interior and surface of the holes, thus obtaining a better firmness of medicines.

The said method for preparation of the medicament eluting apparatus according to the present invention also comprises preprocessing the surface of the apparatus body prior to the step (1): using ultrasonic wave to clean the surface of the apparatus body with acetone or alcohol, and then drying it.

The said method for preparation of the medicament eluting apparatus according to the present invention, further comprises postprocessing the surface of the apparatus body after the step (2): using ultrasonic wave to clean the processed apparatus body firstly with acetone and then with distilled water, drying the cleaned apparatus body in a dryer or immersing the apparatus body in hydrochloric acid solution prepared with distilled water, and then placing it in a thermostat for 30min to 48h.

The said holes are nano-scale or micron-scale, the mean value of the hole size d and hole depth h varies in the range of 1 nm to 500 µm.

The said micron-scale holes are mono-size or bimodal-size or multimodal-size micron-scale holes, namely, they are micron-scale holes distributed in an even size or in uneven sizes with two or more statistic mean values of the hole size or the hole depth.

The said nano-scale holes are mono-size or bimodal-size or multimodal-size nano-scale holes, namely, they are nano-scale holes distributed in an even size or in uneven sizes with two or more statistic mean values of the hole size or the hole depth.

The said multiple medicines refer to two or more medicines, which are selected from one or more of the group consisting of: medicine therapeutic agents, carrier therapeutic genes and bioactive substances.

The said apparatus body includes stent, catheters, guide wire, cardiac pacemaker, cardiac valve, surgical implant material, hard tissue implants and nonmetal medical apparatus with ceramic, organic polymer, inorganics, metal oxide matrix; the said stent is non-polymer stent including balloon expanded stent, self-expanded stent, vascular stent and non-vascular stent, stent with medical stainless steel, nickel-titanium shape-memory alloy, cobalt alloy, pure titanium, titanium alloy and tantalum, titanium alloy or gold matrix which has good biocompatibility, and wire-braided, pipe-laser-cutting, mould-casting and welding stent.

The said apparatus body may further comprise an outermost film layer of protein, polysaccharides, medicine therapeutic agents or the like which can be absorbed by an organism.

The said coating can be obtained by firstly coating several medicines and then coating the others on the apparatus body surface.

The said coating can be obtained by firstly coating several medicines and then coating all the multiple medicines on the apparatus body surface.

The said coating can be obtained by firstly coating the mixture of the multiple medicines and then coating several medicines thereof on the apparatus body surface.

The said coating can be obtained by firstly coating several medicines on the inner surface of the apparatus and then coating the other medicines on the rest surface(s) of the apparatus.

The said coating can be obtained by firstly coating the mixture of the multiple medicines on the inner surface of the apparatus and then coating several medicines thereof on the rest surface(s) of the apparatus.

The said coating can be obtained by firstly coating several medicines on part of the surface of the apparatus and then coating the other medicines on the rest surface(s) of the apparatus.

The said coating can be obtained by firstly coating several medicines on upper part of the apparatus and then coating the other medicines or all the medicines on the lower part of the apparatus.

The said coating can be obtained by firstly coating several medicines on the front part of the apparatus and then coating the other medicines or all the medicines on the hind part of the apparatus.

The present invention achieves the following advantageous effects:
1. Polymers are not contained in the apparatus body, and that reduces the risk of long-term thrombosis caused by implantation of the prior drug-containing polymer.
2. Nano-scale holes have no effect on the mechanical properties of the apparatus body, compared to the micron-scale or even visible holes and drug-storing tanks. It has been proved by the animal experiments that, the non-polymeric stent with two medicines are more reliable and effective than the existing polymer drug-eluting stents.
3. The medicament eluting apparatus according to the present invention does not reduce the mechanical properties and the support force and other physical properties of the apparatus body, so as to effectively control the drug release rate and significantly lower the restenosis rate after surgery.
4. The medicament eluting apparatus according to the present invention can be widely used in drug-eluting medical apparatuses, especially when used in vascular stents, it can achieve good effects in treatment of vascular lesions and prevention of restenosis.

### Brief Description of the Figures

Figure 1 shows block diagram 1 of the present invention;
Figure 2 shows block diagram 2 of the present invention;
Figure 3 shows an embodiment according to the present invention: coating a mixture of two medicines on the surface of the apparatus body;
Figure 4 shows an embodiment according to the present invention: firstly coating one of two medicines and then coating the other medicine on the surface of the apparatus body;
Figure 5 shows an embodiment according to the present invention: firstly coating a mixture of two medicines and then coating a controlled release layer on the surface of the apparatus body;
Figure 6 shows an embodiment according to the present invention: half part of the longitudinal section of the stent is coated with one medicine, and the rest part is coated with another medicine;
Figure 7 shows an embodiment according to the present invention: half part of the horizontal section of the stent is coated with one medicine, the rest part is coated with another medicine, and then a controlled release layer is coated on the surface;
Figure 8 shows an embodiment according to the present invention: half part of the horizontal section of the stent is coated with one medicine, and the rest part is coated with another medicine.

### Specific Mode for Carrying out the Invention

The following examples are given by way of illustration only and are not intended to limit the scope of the invention.

### Example 1

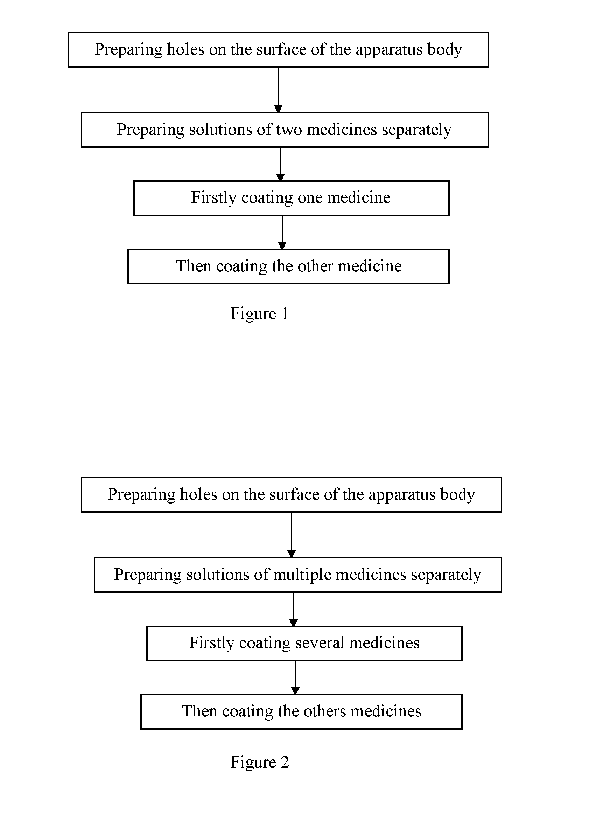

As is shown in figure 1, using H-S stainless steel stent as the apparatus body, the nano-scale hole structure releasing two medicines is prepared, and the process comprises the steps of:
(1) pretreating the surface of the apparatus body: washing the surface of the apparatus body with 99.5% acetone to remove impurities, cleaning the apparatus body under 50khz ultrasonic wave for 10min to remove impurities on the surface of the apparatus body; placing the cleaned apparatus body in a dryer for 60min at the temperature of 30 to 40°C, and then storing it for subsequent use;
(2) preparing holes, including mono-size nano-scale holes and multimodal-size nano-scale holes, and the process comprises the steps of:
   a. preparing mono-size nano-scale holes directly on the apparatus body raw material by the method of corrosion with acid solution to form the holes or anodic oxidation, and the process comprises the step of:
      immersing the apparatus into hydrochloric acid with a 38% concentration as the corrosion solution for 30 minutes at 80°C to form mono-size holes with the hole size of about 400 nm on the surface of the apparatus body;
   b. preparing the mono-size nano-scale holes directly on the surface of the apparatus body by using the method of corrosion with acid solution to form the holes, and then using anodic oxidation or the combination of microarc oxidation and microarc nitridation to form multimodal-size nano-scale complex holes. Specifically, the process of anodic oxidation method comprises the steps of: connecting the apparatus body as a anode with the anode of a pulse power supply, connecting the aluminum sheets as the cathode with the cathode of a pulse power supply, placing the apparatus body and the cathode metal sheets in hydrochloric acid solution with a concentration of 20% simultaneously, the current is set to 5A, the frequency is 1000 Hz, and the time is 15 min, thereby the bimodal-size nano-scale holes can be obtained on the surface of the apparatus body;
(3) preparing medicine solutions of taxol and probucol respectively: dissolving 10mg of taxol and 10mg of probucol in 100ml of ethanol separately, shaking up the solution until the medicine is fully dissolved, and then placing the solution in the refrigerator for subsequent use;
(4) spraying the mixed solution: installing the apparatus in the spraying machine, uniformly spraying the prepared medicines solution (firstly coating probucol solution and then coating taxol solution after the solvent is volatilized) into the holes of the surface of the apparatus body in turn, wherein, some of the medicines are adhered to the surface of the apparatus body;
(5) post-processing the surface of the apparatus body: putting the coated apparatus body in 99.5% acetone solution (analytically pure), then cleaning the apparatus body with distilled water under ultrasonic wave at a frequency of 100 kHz for 10min, respectively; finally, putting the apparatus body in the dryer and setting the temperature at 30-40°C, after drying out for 30min, forming the holes with size of 100-1000nm.

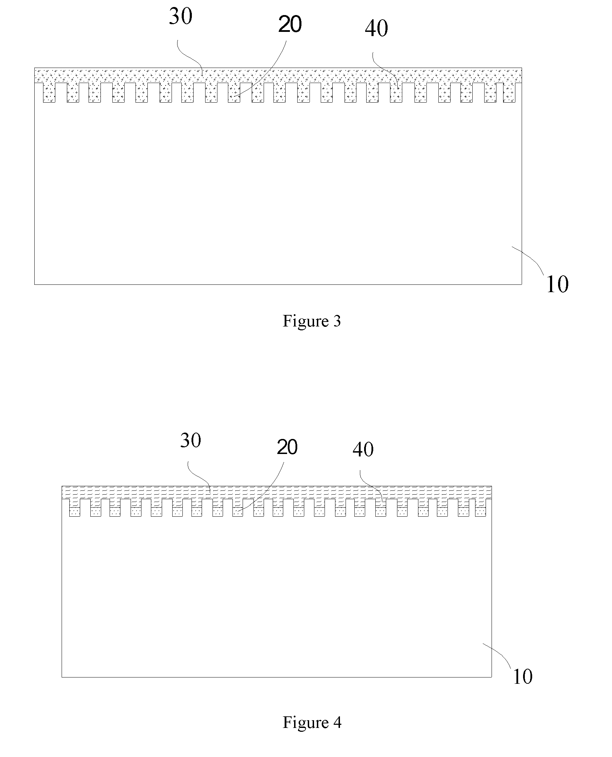

As is shown in figure 4, the said nano-scale holes structure capable of releasing two medicines includes apparatus body 10, active medicines 20 and 30 coated in turn, and holes 40; the said holes 40 represent a large number of nano-scale holes without any compartment between them and the apparatus body 10. The nano-scale holes 40 are medicine-carrying slot or hole structure. Active medicine 20 is firstly coated on part of holes 40 and then the active medicine 30 is coated at the same area. Part of active medicine 30 is loaded in nano-scale holes 40 with individual size and the other active medicine 30 is adhered to the surface of the apparatus body 10.

### Example 2

As is shown in figure 2, using AZ31B magnesium alloy stent as the apparatus body, the nano-scale holes structure releasing multiple medicines is prepared, and the process comprises the steps of:
(1) preparing holes on the surface of the apparatus body: forming holes with size of 100nm ~ 500nm according to the specific method as shown in example 1;
(2) preparing the solution of two medicines rapamycin and probucol: dissolving 10mg of rapamycin and 5mg of probucol in 100ml of acetone separately, shaking up the solution until the medicines are fully dissolved, and then placing the solution in the refrigerator for subsequent use;
(3) coating the two medicines on the surface of the apparatus body simultaneously: coating rapamycin and probucol on the surface of the apparatus body by dipping;
(4) further coating a controlled release layer of heparin: dissolving 1 mg of heparin in 20 ml of saline, and shaking up the solution to make it dissolve completely to prepare heparin solution for subsequent use;
(5) dipping the stent coated with two medicines in the heparin solution for 1 min, and then vaporizing the solution.

After the layer of mixed medicine is dried, a layer of polysaccharides (1 mg of polysaccharides is dissolved in 10 ml of saline to prepare a solution) is further coated on the surface of the apparatus body, and the coating process can be carried out by spraying, so as to control the release rate of the mixed medicines and prevent the loss during the delivery process of the medicines.

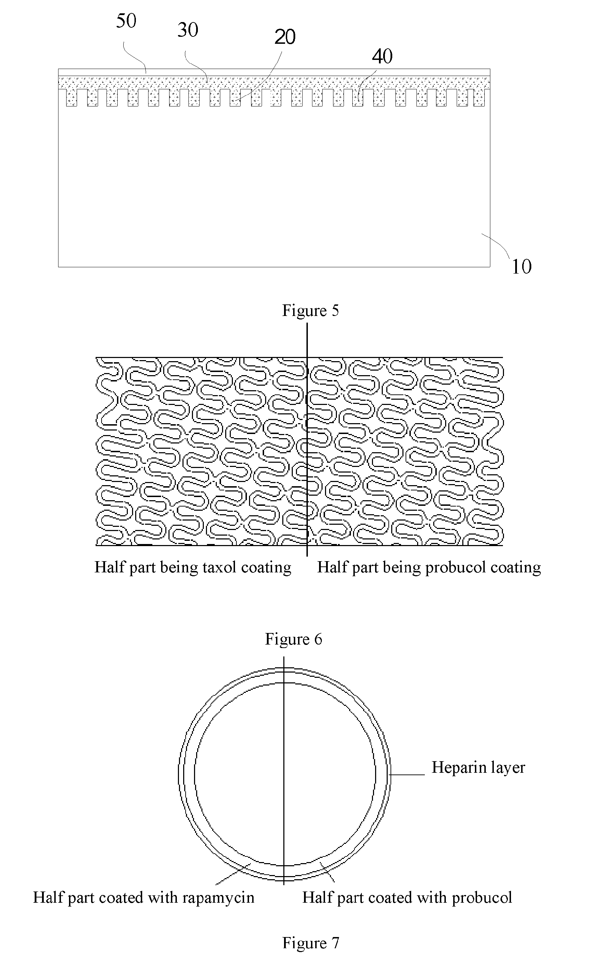

As is shown in figure 5, the said nano-scale holes structure capable of releasing multiple medicines includes apparatus body 10, active medicines 20 and 30, and holes 40; the said holes 40 represent a large number of nano-scale holes, which are directly formed on the apparatus body raw material by chemical or physical methods such as corrosion, anodic oxidation, microarc oxidation and microarc nitridation or combinations thereof, and no compartment exists between the nano-scale holes 40 and apparatus body 10. The nano-scale holes 40 are medicine-carrying slot or hole structure; the mixture of the active medicine 20 and 30 is loaded in nano-scale holes 40 with individual size and partly adhered to the surface of the apparatus body 10, finally, a control layer 50 is coated on the outside of the layer of two medicines.

### Example 3

As is shown in figure 2, using AZ31B magnesium alloy stent as the apparatus body, the nano-scale hole structure releasing multiple medicines release structure is prepared according to the method as shown in example 2, and the process comprises the step of:
preparing the solution of two medicines taxol and probucol: dissolving 10mg of taxol and 5mg of probucol in 100ml of acetone separately, shaking up the solution until the medicines are fully dissolved, and then placing them in the refrigerator for subsequent use;
   (3) coating the two medicines on the surface of the apparatus body, and then coating a layer of taxol again by dipping after the above coated medicines drying;
   (4) further coating a controlled release layer of polysaccharides:

After the layer of mixed medicines is dried, a layer of polysaccharides (1 mg of polysaccharides is dissolved in 10 ml of saline to prepare a solution) is further coated on the surface of the apparatus body, and the coating can be carried out by spraying, so as to control the release rate of the mixed medicines, and prevent the loss during the delivery process of the medicines.

### Example 4

Using pure titanium stent as the apparatus body, the nano-scale hole structure releasing two medicines is prepared by coating the mixed solutions of the two medicines simultaneously in the micro-holes and on the surface of the pure titanium stent with nano-scale micro-holes, and the process comprises the steps of:
(1) preparing holes on the surface of the apparatus body: preparing nano-scale hole structure releasing two medicines , and forming the holes with size of 100-1000nm according to the method as shown in example 1;
(2) preparing the mixed solution of two medicines taxol and probucol: dissolving 10mg of taxol and 10mg of probucol in 100ml of ethanol separately, shaking up the solution until the medicines are fully dissolved, and then placing them in the refrigerator for subsequent use;
(3) coating the two medicines on the surface of the apparatus body simultaneously: coating the mixed solution of taxol and probucol on the surface of the apparatus body by spraying, and volatilizing the solution.

As is shown in figure 3, a medicament eluting apparatus with nano-scale holes structure capable of releasing two medicines includes apparatus body 10, active medicines 20 and 30, and holes 40; the said holes 40 represent a large number of nano-scale holes, which are directly formed on the apparatus body raw material by chemical or physical methods such as corrosion, anodic oxidation, microarc oxidation and microarc nitridation or combinations thereof, and no compartment exists between the nano-scale holes 40 and apparatus body 10; the mixture of the active medicine 20 and 30 is loaded in the nano-scale holes 40 with individual size and partly adhered to the surface of the apparatus body 10.

### Example 5

Using nickel-titanium alloy stent as the apparatus body, the nano-scale hole structure releasing two medicines is prepared according to the method as shown in example 1. Figure 6 shows the longitudinal section view of the stent. After the holes on the surface of the stent are prepared, solutions of two medicines are prepared respectively by dissolving 10mg of taxol or 10mg of probucol in 100ml of tetrahydrofuran separately. Probucol is coated on the upper half part of the longitudinal direction by dipping, after volatilizing the solution, taxol is coated on the rest half part by spraying, thus the non-polymeric medicament eluting nickel-titanium alloy stent carrying two medicines with micro-holes is prepared.

### Example 6

Using titanium alloy stent as the apparatus body, the nano-scale hole structure releasing multiple medicines is prepared according to the method as shown in example 1. Figure 7 shows the longitudinal section view of the stent. After the holes on the surface of the stent are prepared, solutions of three medicines are prepared by dissolving 10mg of rapamycin in 100ml of tetrahydrofuran, 10mg of probucol in 100ml of tetrahydrofuran, and 10mg of heparin in 20ml of saline separately, shaking up the solutions until the medicines are fully dissolved. Probucol is coated on the upper half part at the horizontal direction by dipping, after the probucol drying, rapamycin is coated on the rest half part by spraying, and after they are both dried, heparin solution is sprayed on the surface, drying under vacuum is performed to prepare a non-polymeric medicament eluting stent carrying two medicines with micro-holes.

### Example 7

The nano-scale hole structure releasing three medicines is prepared by using cobalt alloy stent as the apparatus body, the mixed solution of the three medicines is simultaneously coated in the micro-holes and on the surface of the cobalt alloy stent with nano-scale micro-holes, and the process comprises the steps of:
(1) preparing holes on the surface of the apparatus body: preparing holes according to the method as shown in example 2, and forming the holes with size of 100-1000nm;
(2) preparing the mixed solution of two medicines taxol and probucol: dissolving 10mg of taxol and 10mg of probucol in 100ml of ethanol separately, shaking up until the medicines are fully dissolved, then placing them in the refrigerator for subsequent use;
(3) coating the two medicines on the surface of the apparatus body simultaneously: coating the solution of taxol and probucol on the surface of the apparatus body by spraying, and volatilizing the solution;
(4) preparing the heparin solution by dissolving 10mg of heparin in 20ml of saline, shaking up until the medicine is fully dissolved, and then placing the solution in the refrigerator for subsequent use;
(5) dipping the stent coated with two medicines in the heparin solution for 1 min, and then vaporizing the solution.

### Example 8

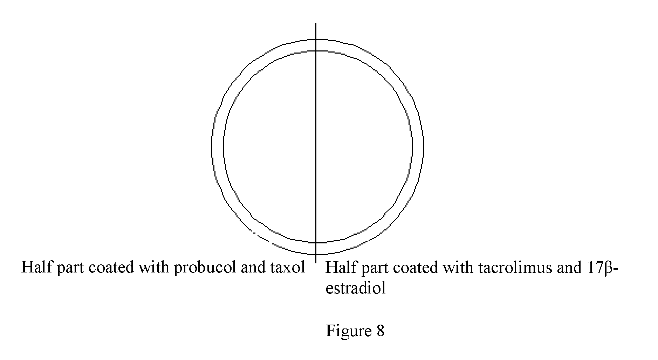

The nano-scale hole structure releasing four medicines is prepared by using stainless steel stent as the apparatus body. Figure 8 shows the longitudinal section view of the stent. After the holes are prepared on the surface of the stent, a solution of two medicines is prepared, for example, by dissolving 10mg of taxol and 10mg of probucol in 100ml of tetrahydrofuran to prepare a mixed solution 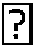 ; by dissolving 10mg of tacrolimus and 10 mg of 17β-estradiol in 100ml of tetrahydrofuran to prepare a mixed solution 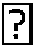 . The mixed solution ① is coated on the upper half part at the horizontal direction by dipping, and then the mixed solution ② is coated on the rest half part by spraying, thus a non-polymeric medicament eluting stent carrying four medicines with micro-holes is prepared.

### Example 9 Implantation Experiment on Animals

Taking into account the intended use of the stents, to ensure the maximal compatibility to the human condition, the closest animal models to human heart_ healthy miniature pigs for experiments (provided by Beijing Agricultural University Improved Variety Plant) were chosen to carry out the performance evaluation experiment of the stent *in vivo*. All the stents were implanted into the anterior descending coronary artery and the circumflex artery of healthy miniature pigs at a ratio of 1.1-1.25:1(stent/ artery), 28 days after implantation, angiography was performed on all the experimental pigs and intravascular ultrasound IVUS is performed on part of the experimental pigs, so as to observe in-stent intimal hyperplasia and restenosis. The following table 1 shows the results of the statistic analysis of the three stents after 28 days implantation through QCA analysis:

**Table 1 The effects of the nano-scale hole stents with multiple medicines**

| Stent type | Stainless steel bare stent ( 12 ) | (Single medicine stent) taxol-containing polymeric stent ( 12 ) | Nano-scale micro-hole stent with multiple medicines ( 12 ) |
|---|---|---|---|
| Mean value of lumen loss (mm) | 1.35 | 0.5 | 0.3 |
| The average degree of stenosis (%) | 45 | 30 | 18 |
| Restenosis rate (%) | 45.46 | 16.67 | 3.50 |

| | | | |
|---|---|---|---|
| Note: For the single medicine stent, the concentration of taxol is 1.0µg/mm²; for the nano-scale micro-hole stent with multiple medicines containing taxol and probucol, the total content of the medicines is 2.0µg/mm², in which taxol is 1.0µg/mm² and probucol is 1.0µg/mm². | | | |

The results of angiography and IVUS on experimental pigs for 28 days showed that, non-polymeric two-medicine eluting stents with nano-scale holes and polymer drug-eluting stent performed better in restenosis rate and lumen loss than stainless steel bare stent, specifically, the restenosis rate and lumen loss of bare stent were higher than those of medicine eluting stent, the restenosis rate and lumen loss of nano-scale hole stent with two medicines were lower than those of polymer stent, it indicated that non-polymeric two-medicine eluting stents with nano-scale holes are more reliable and effective in reducing restenosis rate of the nano-scale holes stent than medicine-containing polymer-coated stent.

The medicament eluting apparatus according to the present invention can reduce the risk of thrombosis caused by implanting the apparatus that uses polymer to carry medicines into human tissue, and improve the effectiveness of the existing single medicine micro-hole apparatus capable of releasing medicines, thus significantly reducing restenosis rates and the late lumen loss after surgery.

### Industrial Applicability

This invention discloses a medicament eluting apparatus with micro-hole structure capable of storing and releasing multiple medicines and preparation method thereof. The said medicament eluting apparatus includes an apparatus body and several nano-scale micro-hole on the apparatus body capable of storing and releasing multiple medicines. On one hand, it can decrease the risk of thrombosis caused by implanting the apparatus that use polymer to carry medicines into human tissue. On the other hand, the synergistic effect of multiple medicines can improve the effectiveness of the medicine eluting micro-hole apparatus capable of releasing medicines, thus significantly reducing restenosis rates and the late lumen loss after surgery.

## Claims

**1.** A medicament eluting apparatus with a micro-hole structure capable of storing and releasing multiple medicines, including an apparatus body and a hole structure for releasing medicines; in which the hole structure for releasing medicines refers to a number of holes on the apparatus body and multiple medicines provided in the holes and on the surface of the apparatus body; in which the said medicines are selected from the group consisting of medicine therapeutic agents, carrier therapeutic genes and bioactive substances; the holes are in nano-scale or micron-scale, and the statistical mean values of the hole size **d** and hole depth **h** vary in the range of 1 nm to 500 µm.

**2.** The medicament eluting apparatus according to claim 1, wherein the medicines contain taxol and/or probucol.

**3.** The medicament eluting apparatus according to claim 1, wherein the said holes are mono-size or bimodal-size or multimodal-size holes in nano-scale, that is, with a number of **n**, the holes are nano-scale holes distributed in even size, or the holes are nano-scale holes distributed in uneven size and have two or more statistical mean values of the hole size or the hole depth.

**4.** The medicament eluting apparatus according to any one of claims 1 to 3, wherein the holes are present in opening, semi-opening, closed, separated, inter-connected, inter-embedded type, or nested type in which a large hole comprises small holes.

**5.** The medicament eluting apparatus according to claim 1, wherein the apparatus body further comprises film layer(s).

**6.** The medicament eluting apparatus according to claim 2, wherein the medicines comprise one or more medicine therapeutic agents selected from the group consisting of heparin, aspirin, hirudin, colchicine, antiplatelet GPIIb/IIIa receptor antagonist, methotrexate, purines, miazines, plant alkaloids and epothilones, series compounds of Tripterygium *Wilfordii*, antibiotics, hormones, antibody medicines for cancer treatment, cyclosporin, tacrolimus and homologues thereof, 15-deoxyspergualin, mycophenolate mofetil, rapamycin and derivatives thereof, FR 900520, FR 900523, daclizumab, depsidomycin, kanglemycin C , spergualin, prodigiosin 25-c, tranilast, myriocin, FR 651814, SDZ214-104, cyclosporine C, bredinin, mycophenolic acid, brefeldin A, WS9482, glucocorticosteroid, tirofiban, abciximab, eptifibatide, actinomycin-D, As₂O₃, 17β-estradiol, trapidil, hexadecadrol and probucol derivatives.

**7.** The medicament eluting apparatus according to claim 1, wherein the carrier therapeutic genes are selected from one or more of the group consisting of cell, virus, DNA, RNA, virus carrier and nonviral carrier.

**8.** The medicament eluting apparatus according to claim 1, wherein the bioactive substances are selected from one or more of the group consisting of cell, yeast, bacteria, protein, peptide and hormone.

**9.** The medicament eluting apparatus according to any one of claims 1, 2 and 5, wherein the medicines further contain rapamycin.

**10.** The medicament eluting apparatus according to any one of claims 1, 2, 5 and 9, wherein the medicines further contain heparin.

**11.** The medicament eluting apparatus according to any one of claims 1, 9 and 10, wherein the medicines further contain tacrolimus.

**12.** The medicament eluting apparatus according to any one of claims 1, 9, 10 and 11, wherein the medicines further contain β- estradiol.

**13.** The medicament eluting apparatus according to claims 1 or 2, wherein the medicines are taxol and probucol.

**14.** The medicament eluting apparatus according to claim 1, wherein the medicines are probucol, rapamycin and heparin.

**15.** The medicament eluting apparatus according to claim 1, wherein the medicines are taxol, probucol, tacrolimus and β- estradiol.

**16.** The medicament eluting apparatus according to any one of claims 1 to 15, wherein the medicines provided in the holes and on the surface of the apparatus body are obtained by coating a mixture of multiple medicines on the surface of the apparatus body, or firstly coating several medicines and then coating other medicines on the surface of the apparatus body.

**16.** The medicament eluting apparatus according to any one of claims 1 to 15, wherein the medicines provided in the holes and on the surface of the apparatus body are obtained by firstly coating several medicines and then coating all the medicines on the surface of the apparatus body, or firstly coating the mixture of multiple medicines and then coating several medicines thereof on the surface of the apparatus body.

**17.** The medicament eluting apparatus according to any one of claims 1 to 15, wherein the medicines provided in the holes and on the surface of the apparatus body are obtained by firstly coating several medicines on the inner surface of the apparatus body and then coating other medicines on the rest surface(s) of the apparatus body, or firstly coating all the medicines on the inner surface of the apparatus body and then coating several medicines thereof on the rest surface(s) of the apparatus body.

**18.** The medicament eluting apparatus according to any one of claims 1 to 15, wherein the medicines provided in the holes and on the surface of the apparatus body are obtained by firstly coating several medicines on the upper part of the apparatus and then coating other medicines or all the medicines or several medicines thereof on the lower part of the apparatus; or firstly coating several medicines on the front part of the apparatus and then coating other medicines or all the medicines or several medicines thereof on the hind part of the apparatus; or firstly coating several medicines on part of the apparatus surface and then coating other medicines on the rest part of the apparatus surface.

**19.** The medicament eluting apparatus according to any one of claims 1 to 15, wherein said coating process is selected from one or more of the group consisting of spraying, dipping and dipping in vacuum.

**20.** The medicament eluting apparatus according to claim 1, wherein the apparatus body includes stent, catheters, guide wire, cardiac pacemaker, cardiac valve, surgical implant material, hard tissue implants and nonmetal medical apparatus with ceramic, organic polymer, inorganics, metal oxide matrix.

**21.** The medicament eluting apparatus according to claim 20, wherein the stent is non-polymer stent including balloon expanded stent, self-expanded stent, vascular stent, non-vascular stent, stents with medical stainless steel, nickel-titanium shape-memory alloy, cobalt alloy, pure titanium, titanium alloy and tantalum, titanium alloy and gold matrix, and wire-braided, pipe-laser-cutting, mould-casting and welding stents.

**22.** A method for preparation of the medicament eluting apparatus according to any one of claims 1 to 21, mainly comprising preparing a hole structure for releasing medicines on the implantable medical apparatus body, and the process including the steps of:
(1) preparing holes on the surface of the apparatus body with one or more coating methods selected from the group consisting of corrosion with acid solution, anodic oxidation, microarc oxidation and microarc nitridation or combinations thereof;
(2) coating multiple medicines in the holes and on the surface of the apparatus body with the process comprising spraying, dipping or dipping in vacuum.

**23.** The method for preparation of the medicament eluting apparatus according to claim 22, comprising dissolving the multiple medicines respectively or mixedly in solvent to formulate pharmaceutical solution prior to the step (2).

**24.** The method for preparation of the medicament eluting apparatus according to claim 22, comprising preprocessing the surface of the apparatus body by cleaning the surface of the apparatus body under ultrasonic wave with acetone or alcohol and drying it prior to the step (1).

**25.** The method for preparation of the medicament eluting apparatus according to claim 22, wherein after the step (2), further comprising postprocessing the surface of the apparatus body by cleaning the processed apparatus body under ultrasonic condition with acetone and distilled water in sequence, drying the cleaned apparatus body in a dryer or immersing the apparatus body in hydrochloric acid solution prepared with distilled water, and then putting it in a thermostat for 30min to 48h.
